(19) Europäisches Patentamt
European Patent Office
Office européen des brevets

(11) Veröffentlichungsnummer: **0 212 221**
**B1**

(12) **EUROPÄISCHE PATENTSCHRIFT**

(45) Veröffentlichungstag der Patentschrift: **09.05.90**

(51) Int. Cl.$^5$: **C 07 C 63/38,** C 07 C 51/265

(21) Anmeldenummer: **86109528.9**

(22) Anmeldetag: **11.07.86**

# (54) Verfahren zur Herstellung von 2,6-Naphthalindicarbonsäure.

(30) Priorität: **16.08.85 DE 3529381**
**12.12.85 DE 3543879**

(43) Veröffentlichungstag der Anmeldung:
**04.03.87 Patentblatt 87/10**

(45) Bekanntmachung des Hinweises auf die Patenterteilung:
**09.05.90 Patentblatt 90/19**

(84) Benannte Vertragsstaaten:
**DE FR GB IT NL**

(56) Entgegenhaltungen:
**DE-A-2 243 158**
**DE-B-1 291 329**
**US-A-3 055 839**

(73) Patentinhaber: **HÜLS AKTIENGESELLSCHAFT**
**Paul-Baumann-Strasse 1**
**D-4370 Marl 1 (DE)**

(72) Erfinder: **Feld, Marcel, Dr.**
**Im Lochgarten 56**
**D-5000 Köln 90 (DE)**

**EP 0 212 221 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft ein Verfahren zur Herstellung von 2,6-Naphthalindicarbonsäure, ausgehend von einem 2-Acyl-6-alkylnaphthalin, vorzugsweise 2-Acetyl-6-methylnaphthalin, durch eine zweistufige Oxidation mit Sauerstoff.

Erfindungsgemäß wird ein 2-Acyl-6-alkylnaphthalin zunächst in einer ersten Oxidationsstufe unter der katalytischen Wirkung einer Manganverbindung in einer aliphatischen Carbonsäure, vorzugsweise Essigsäure, als Lösungsmittel bzw. in einem eine aliphatische Carbonsäure enthaltenden Lösungsmittel mit Sauerstoff oder einem Sauerstoff enthaltenden Gas, vorzugsweise Luft, oxidiert und das dabei gebildete Oxidationsprodukt in einer zweiten Oxidationsstufe, nunmehr in Gegenwart einer Kobalt- und einer Bromverbindung als Katalysator, in einer aliphatischen Carbonssäure als Lösungsmittel bzw. einem eine aliphatische Carbonsäure, vorzugsweise Essigsäure, enthaltenden Lösungsmittelgemisch mit Sauerstoff bzw. einem Sauerstoff enthaltenden Gas, vorzugsweise Luft, zu 2,6-Naphthalindicarbonsäure oxidiert.

Gegenstand der Erfindung ist ein Verfahren zur Herstellung von 2,6-Naphthalindicarbonsäure, durch Oxidation in flüssiger Phase mit Sauerstoff bzw. einem Sauerstoff enthaltenden Gas, dadurch gekennzeichnet, daß das 2-Acyl-6-alkylnaphthalin in einer ersten Oxidationsstufe in Gegenwart einer im Reaktionsgemisch löslichen Manganverbindung als Katalysator in einer aliphatischen Carbonsäure als Lösungsmittel bzw. in einem eine aliphatische Carbonsäure enthaltenden Lösungsmittelgemisch bei Temperaturen von 80 bis 180°C oxidiert wird und die entstehende 6-Alkyl-2-naphthoesäure in einer zweiten Oxidationsstufe in Gegenwart einer im Reaktionsgemisch löslichen Kobaltverbindung und einer Bromionen liefernden Komponente als Katalysator in einer aliphatischen Carbonsäure als Lösungsmittel bzw. in einem eine aliphatische Carbonsäure enthaltenden Lösungsmittelgemisch bei einer Reaktionstemperatur von 150 bis 250°C zum Produkt oxidiert wird.

Der entscheidende Unterschied zwischen der ersten und zweiten Oxidationsstufe des erfindungsgemäßen Verfahrens betrifft also die verwendeten Katalysatoren. In der ersten Oxidationsstufe ist ein Mangankatalysator erforderlich. In der zweiten Oxidationsstufe ist dagegen eine Kobalt- und eine Bromkomponente als Katalysator erforderlich. Eine Manganverbindung kann bei der zweiten Oxidationsstufe zusätzlich zu der Kobalt- und Bromverbindung eingesetzt werden, jedoch ist das nicht notwendig.

Es sind bereits mehrere Verfahren zur Herstellung der für die Gewinnung spezieller Polymerer geeigneten 2,6-Naphthalindicarbonsäure bekannt. Die Mehrzahl der Verfahren geht von 2,6-Dialkylnaphthalin, insbesondere von 2,6-Dimethylnaphthalin, aus. Die Bereitung der Ausgangsprodukte bereitet in der notwendigen Isomerenreinheit allerdings erhebliche Schwierigkeiten. Auch sind die Ergebnisse der Oxidationen mit Luftsauerstoff bezüglich der Ausbeute und Reionheit des Zielproduktes bei der Mehrzahl der beschriebenen Verfahren gemäß DE—OS 21 07 357, Japan Kokai 76 06.953, Japan Kokai 77 17.453 oder der Belg. PS 660 333 unbefriedigend.

Beim erfindungsgemäßen Verfahren wird kein Dialkylnaphthalin, sondern ein durch Acylierung eines Alkylnaphthalin zugängliches 2-Acyl-6-alkylnaphthalin, insbesondere das 2-Acetyl-6-methylnaphthalin, als Ausgangsprodukt verwendet.

Wie die Vergleichsbeispiele A und B zeigen, sind für die Oxidation derartiger Verbindungen mit Luftsauerstoff in essigsaurer Lösung die für die Oxidation von Alkylaromaten, beispielsweise auch von Dialkylnaphthalinen, üblichten, durch eine Kombination einer Kobalt- und einer Bromverbindung als Katalysator charakterisier en Bedingungen wenig geeignet. Vielmehr sind die erfindungsgemäßen Oxidationsbedingungen erforderlich, um ausgehend von einem 2-Acyl-6-alkylnaphthalin mit überlegenen Ergebnissen 2,6-Naphthalindicarbonsäure herzustellen.

Geeignete Ausgangsstoffe für die Herstellung von 2,6-Naphthalindicarbonsäure nach dem erfindungsgemäßen Verfahren sind 2-Acyl-6-alkylnaphthaline der allgemeinen Formel I:

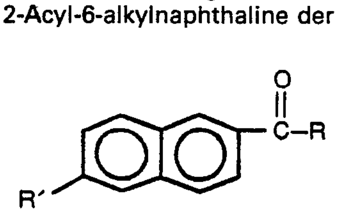

(I)

Darin haben R und R′ die Bedeutung gleicher oder unterschiedlicher, geradkettiger oder verzweigter Alkylgruppen mit 1 bis 6 C-Atomen, wobei das in R′dem aromatischen Rest bzw. das in R der Carbonylgruppe nächststehende C-Atom zumindest noch 1 H-Atom tragen muß. Bevorzugtes Ausgangsdprodukt ist das 2-Acetyl-6-methylnaphthalin (R = R′ = $CH_3$). Die Ausgangsstoffe sind durch Friedel-Crafts-Acylierung aus Alkyl-, besonders 2-Methylnaphthalin, zugängig.

Charakteristisch für das erfindungsgemäße Verfahren ist, daß es zwei, bezüglich der Bedingungen sehr unterschiedliche Oxidationsstufen beeinhaltet. Die erste Oxidations stufe wird durch eine Manganverbindung bei Abwesenheit einer Kobalt- und/oder Bromverbindung katalysiert. Die bevorzugte Konzentration des Mn-Katalysators beträgt pro Gew.-Teil des Acyl-alkylnaphthalins 0,001 bis 0,1,

vorzugsweise 0,01 bis 0,05 Gew.-Teile Mangan in Form einer im Reaktionsgemisch löslichen Manganverbindung, beispielsweise Mangan (II)-acetat oder Mangan (II)-acetylacetonat. Neben dem Mangankatalysator kann in der ersten Oxidationsstufe gegebenenfalls die Kobalt — der Bromkomponente in der für die zweite Oxidationsstufe geeigneten Konzentration anwesend sein, jedoch nicht beide Komponenten gleichzeitig. Bevorzugt wird jedoch ein Mangankatalysator in Abwesenheit einer Kobalt- und einer Bromkomponente.

Geeignete Lösungsmittel für die erfindungsgemäße Oxidation von Acyl-alkylnaphthalin sind aliphatische Carbonsäuren mit vorzugsweise 2 bis 4 C-Atomen, insbesondere Essigsäure und/oder die Anhydride der betreffenden aliphatischen Carbonsäuren, insbesondere Acetanhydrid. Die aliphatischen Carbonsäuren oder die entsprechenden Anhydride können allein, in Kombination untereinander oder auch in Kombination mit einem weiteren, unter den Reaktionsbedingungen gegen Sauerstoff beständigen Lösungsmittel eingesetzt werden. Ein gut geeignetes Lösungsmittel ist neben reiner Essigsäure auch wässrig-verdünnte Essigsäure mit beispielsweise 1 bis 20 Gew.-% Wasser.

Das Acyl-alkylnaphthalin und das Lösungsmittel bzw. Lösungsmittelgemisch werden beim erfindungsgemäßen Verfahren vorzugsweise in Gewichtsverhältnissen von ca. 1:2 bis 1:20, insbesondere 1:3 und 1:10 eingesetzt.

Das in der ersten Oxidationsstufe erhaltene Reaktionsgemisch kann, ggf. nach destillativer Abtrennung von Leichtsiedern wie Wasser und/oder Ameisensäure oder nach Zusatz von weiterem Lösungsmittel, ohne Isolierung des aus dem Acyl-alkylnaphthalin entstandenen Reaktionsproduktes in der zweiten Oxidationsstufe nach Zugabe einer geeigneten Kobalt- und einer Bromverbindung weiter oxidiert werden. Vorzugsweise wird das in der ersten Oxidationsstufe aus dem Acylalkylnaphthalin entstandene Reaktionsprodukt, ggf. nach vorheriger Einengung oder aber Verdünnung des Reaktionsgemisches, beispielsweise durch Wasserzusatzt, durch eine übliche Fest-Flüssig-Trennung isoliert, bevor es zur zweiten Oxidationsstufe eingesetzt wird. Dann kann die bei der Produktisolierung erhaltene Mutterlauge erneut als Reaktionsmedium für die Oxidation des Acyl-alkylnaphthalins verwendet werden. Dadurch, besonders bei mehrfacher Wiederverwendung der Mutterlauge, wird die Ausbeute beträchtlich erhöht. Zweckmäßig wird zuvor Wasser und andere Leichtsieder entfernt und ggf. Essigsäure und der Katalysator ergänzt.

Die erste Oxidationsstufe des Verfahrens erfolgt bei Temperatur von 80 bis 180°C, vorzugsweise von 100 bis 160°C. Der Druck beträgt 0 bis 60 bar, vorzugsweise 5 bis 40 bar.

Entscheidend für die erste Oxidationsstufe des erfindungsgemäßen Verfahrens ist die Verwendung des Mangankatalysators.

Die zweite Oxidationsstufe ist dagegen durch die Verwendung einer Katalysatorkombination aus einer Kobalt- und einer Bromverbindung gekennzeichnet. Geeignete Kobaltverbindungen sind insbesondere Kobaltbromid, Kobaltacetat oder eine in essigsaurer Lösung Kobaltacetat liefernde Verbindung. Geeignete Bromkomponenten sind neben dem Kobaltbromid auch elementares Brom, Bromwasserstoff, Acetylbromid und insbesondere Alkalibromide oder Ammoniumbromid. Entscheidend ist, daß die Bromkomponente unter den Reaktionsbedingungen Bromatome oder Bromionen zu bilden vermag.

Die geeignete Katalysatorkonzentration beträgt pro Gew.-Teil des in der ersten Oxidationsstufe eingesetzten Acyl-alkylnaphthalins bzw. des entstandenen und ggf. isolierten und in der zweiten Oxidationsstufe eingesetzten Oxidationsproduktes 0,0005 bis 0,05, vorzugsweise 0,002 bis 0,02 Gew.-Teile Kobalt in Form einer im Reaktionsgemisch löslichen Kobaltverbindung und 0,001 bis 0,1, vorzugsweise 0,002 bis 0,04 Gew.-Teile Brom in Form einer geeigneten Bromverbindung. Mangan stört nicht den Reaktionsverlauf der zweiten Stufe.

Bevorzugtes Lösungsmittel für die zweite Oxidationsstufe ist allerdings ebenfalls Essigsäure bzw. eine wässrig-verdünnte Essigsäure. Geeignete Konzentrationsverhältnisse sind ca. 2 bis 50, vorzugsweise 4 bis 20 Gew.-Teile Essigsäure pro Gew.-Teil des zu oxidierenden Produktes.

Die Reaktionstemperatur der zweiten Oxidationsstufe liegt bei 150 bis 250°C, vorzugsweise bei 160 bis 220°C. Die Temperatur lieft vorzugsweise um 20 bis 80°C, besonders 30 bis 50°C höher als in der ersten Stufe. Der Druck liegt bei 5 bis 80 bar, vorzugsweise bei 10 bis 60 bar.

Die nach der zweiten Oxidationsstufe resultierende 2,6-Naphthalindicarbonssäure kann aus dem Reaktionsgemisch durch eine übliche Fest-Flüssig-Trennung isoliert werden.

Mutterlaugen der zweiten Oxidationsstufe können in entsprechender Weise für eine erneute Oxidation der zweiten Stufe wiederverwendet werden, wodurch die Ausbeuten steigen, der Katalysatorbedarf sinkt und der Aufwand für die Aufarbeitung der Mutterlaugen sehr vermindert ist.

Dieses zweistufige Verfahren ergibt gegenüber einstufigen Verfahren eine erhebliche Verbesserung der Reinheit des Zielproduktes. Allerdings sind für hohe Reinheitsgrade von über 97% große Lösungsmittelmengen in der zweiten Oxidationsstufe von beispielsweise 13 bis 50 Gew.-Teilen pro Gew.-Teil Alkylnaphthoesäure erforderlich. Derart hohe Verdünnungsgrade sind für ein technisches Verfahren aber dann schwierig, wenn große Filtratmengen zum Wiedereinsatz als Reaktionsmedium oder zur Rückgewinnung der Essigsäure destillativ aufzuarbeiten sind. Der Reaktor muß bei einer großen Lösungsmittelmenge groß sein bzw. sind in gleichen Zeiten geringere Mengen herstellbar.

Es zeigte sich weiterhin, daß die Reinheit von bei sonst gleichen Bedinungen gewonnener 2,6-Naphthalindicarbonsäure stark bei vermindertem Anteil des Lösungsmittels absinkt. So resultierte bei einem Gewichtsverhältnis des Lösungsmittels Essigsäure zu der Methylnaphthoesäure von 12:1 im

Einsatzgemisch das Zielprodukt nur mit einer Reinheit von 90,3%. Als Hauptverunreinigung enthielt das Oxidationsprodukt noch 6-Formyl-2-naphthoesäure und den Ausgangsstoff 6-Methyl-2-naphthoesäure. Bei weiterer Erhöhung der Einsatzmenge an 6-Methyl-2-naphthoesäure wurde die 2,6-Naphthalindicarbonsäure nur mit einer Reinheit von 87% erhalten.

2,6-Naphthalindicarbonsäure sollte nun im möglichst hoher Reinheit durch Oxidation von 6-Alkyl-2-naphthoesäure bei möglichst hohem Einsatzverhältnis von 6-Alkyl-2-naphthoesäure zu der als Lösungsmittel verwendeten aliphatischen Carbonsäure hergestellt werden.

In weiterer Ausbildung der Erfindung wird daher 2,6-Naphthalindicarbonsäure dadurch hergestellt, daß die zu oxidierende Alkylnaphthoesäure dem Reaktionsgemisch ganz oder zum größten Teil während der Reaktion zugesetzt wird. Dadurch wird bei vergleichbarer oder besserer Reinheit des Zielproduktes in der gleichen Menge des Lösungsmittels dann eine erheblich größere, gar vielfache Menge einer 6-Alkyl-2-naphthoesäure oxidiert, wenn diese nicht insgesamt im Einsatzgemisch vorgelegt, sondern größtenteils erst während der Reaktion zugesetzt wird. Diese Eindosierung kann portionsweise oder vorteilhaft kontinuierlich erfolgen und annähernd der Reaktionsgeschwindigkeit angepaßt werden.

Auch die erste Stufe kann so ausgeführt werden. Die Zugabe der Alkylnaphthoesäure kann in fester Form, oder aber suspendiert oder gelöst in einem geeigneten, oxidationsbeständigen Lösungsmittel, vorzugsweise in der als Reaktionsmedium verwendeten aliphatischen Carbonsäure mit 2 bis 4 C-Atomen erfolgen.

Bei Zudosierung einer Lösung der Alkylnaphthoesäure, z.B. in Essigsäure und/oder Acetanhydrid, kann die Lösung bei erhöhter Temperatur, beispielsweise bei Reaktionstemperatur, hergestellt und zugegeben werden. Bevorzugt sind jedoch Suspensionen des Ausgangstoffes im Lösungsmittel bis zur Grenze der Fließfähigkeit.

Die Wirkung der Zugabe möglichst großer Mengen des Ausgangsstoffes je Menge Lösungsmittel kontinuierlich oder in häufigen kleinen Portionen auf die Reinheit ist überraschend hoch und kann dadurch demonstriert werden, daß die Oxidation willkürlich in einzelne Abschnitte mit jeweils zu oxidierenden Teilmengen der vorgegebenen Menge Alkylnaphthoesäure unterteilt wird.

Technisch erfolgt eine kontinuierliche oder annähernd kontinuierliche Zugabe des Ausgangsstoffes nach Maßgabe des Umsatzes in möglichst konzentrierter Suspension, ggf. als Feststoff.

Zum Start der Reaktion ist im Einsatzgemisch eine Teilmenge von 5 bis 10% der insgesamt zu oxidierenden Alkylnaphthoesäure ausreichend, obwohl auch größere Mengen, bis beispielsweise 25%, möglich sind. Zugegebene Einzelportionen sollen nicht größer als 25% sein.

Die Gesamtmenge kann dann unter diesen Bedingungen vorteilhaft 0,1 bis 0,6 Gew.-Teile pro Gew.-Teil Lösungsmittel betragen.

## Beispiel 1

1. Oxidationsstufe:

Ein mit Rührer, Gaseinleitungsrohr, Temperaturfühler, Manometer und Druck-Rückflußkühler ausgestatteter, berheizbarer Autoklav aus Hastelloy C wurde mit 100 g 2-Acetyl-6-methylnaphthalin, 380 g Essigsäure, 20 g Wasser und 15 g Mangan (II)-acetat beschickt. Durch die Lösung wurde bei 140°C und einem Druck von 25 bar unter Rühren Preßluft mit einer Gasaustrittsgeschwindigkeit von 3 l/min. geleitet. Der Reaktionsverlauf wurde durch kontinuierliche Messung des Sauerstoffgehaltes im Abgas kontrolliert. Als dieser Sauerstoffgehalt nach einer Reaktionszeit von 105 min. wieder den Ausgangswert von 21% erreicht hatte, wurde die Lufteinleitung abgestellt, das Reaktionsgemisch unter Rühren auf Raumtemperature abgekühlt, filtriert und der Filterkuchen mit 300 g 95 Gew.-%iger Essigsäure gewaschen. Es wurden 70,3 g 6-Methyl-2-naphthoesäure (69,3% d. Th.) erhalten. Reinheit 99,5%.

Die mit den Waschfiltraten vereinigte Mutterlauge wurde unter Abdestillation von Wasser, Ameisensäure und Essigsäure auf 380 g eingeengt. Nach Zusatz von 20 g Wasser, 67,5 g 2-Acetyl-6-methylnaphthalin und 3,5 g Mangan (II)-acetat wurde erneut unter den zuvor beschriebenen Bedingungen oxidiert. Diesmal wurden 67,3 g 6-Methyl-2-naphthoesäure (98,2%, bezogen auf frisch eingesetztes 2-Acetyl-6-methylnaphthalin) erhalten.

2. Oxidationsstufe:

Analog dem zuvor beschriebenen Versuch für die erste Oxidationsstufe wurden 36,5 g des dort isolierten Produktes in einer Lösung von 2 g Kobaltacetat und 0,5 g Natriumbromid in 500 g Essigsäure bei einer Temperatur von 190°C, einem Druck von 25 bar und einer Gasaustrittsgeschwindigkeit von 4 l/min. oxidiert. Nach einer Reaktionszeit von 140 min. wurde das auf Raumtemperatur abgekühlte Reaktionsgemisch filtriert, der Filterkuchen mit 250 g Essigsäure gewaschen und getrocknet. Es wurden 35,4 g 2,6-Naphthalindicarbonsäure (81,9%) mit einer gaschromatographisch bestimmten Reinheit von 97,9% erhalten.

Die mit dem Waschfiltrat vereinte Mutterlauge wurde unter destillativer Abtrennung von Wasser und Essigsäure auf ein Gewicht von 500 g eingeengt, 0,2 g Natriumbromid und 35 g des nach der ersten Oxidationsstufe isolierten Produktes zugesetzt und dann erneut unter den zuvor beschriebenen Bedingungen oxidiert und aufgearbeitet. Es wurden 36,2 g (86,9% der Theorie) 2,6-Naphthalindicarbonsäure mit einer Reinheit von 97,4% gewonnen.

Beispiel 2

Eine Mischung von 50 g 2-Acetyl-6-methylnaphthalin, 380 g Essigsäure, 20 g Wasser und 7,5 g Mangan (II)-acetat wurde bei 140°C, 25 bar und einem Gasaustritt der Luft von 1,5 l/min. oxidiert. Nach beendeter Sauerstoffaufnahme wurden 5 g Kobaltacetat und 0,5 g Natriumbromid zugefügt und in ensprechender Weise bei 190°C Luft durchgeleitet. Nach dieser 2. Oxidationsstufe wurden durch Aufarbeitung wie im Beispiel 1 50,8 g Produkt mit einem Gehalt von 93% 2,6-Naphthalindicarbonsäure isoliert (80,8% d. Th.)

Vergleichbeispiel A

Entsprechend der in Beispiel 1 beschriebenen Versuchsanordnung wurden 50 g 2-Acetyl-6-methylnaphthalin, 400 g Essigsäure, 5 g Kobaltacetat und 0,5 g Natriumbromid zur Oxidation bei 190°C, einem Druck von 25 bar und einem Gasaustritt von 2 l/min. eingesetzt. Aus dem nach beendeter Sauerstoffaufnahme auf Raumtemperatur angekühlten Reaktionsgemisch wurden durch Filtration 15,2 g eines Produktgemisches mit einem Gehalt von nur 30,9% 2,6-Naphthalindicarbonsäure isoliert.

Vergleichsbeispiel B

Vergleichsbeispiel A wurde bei einer Reaktionstemperatur von 190°C wiederholt. Diesmal wurden aus dem Reaktionsgemisch nur 14,7 g Feststoff mit einem Gehalt von 40% 2,6-Naphthalindicarbonsäure isoliert.

Vergleichsbeispiel C

Vergleichsbeispiel A wurde mit zusätzlich noch 5 g Mangan (II)-acetat als Katalysatorbestandteil wiederholt. Aus dem Reaktionsgemisch wurden 50,0 g eines Produktgemisches mit einem Gehalt von 79,1% 2,6-Naphthalindicarbonsäure isoliert.

Beispiel 3

Die in Beispiel 1 beschriebene 1. Oxidationsstufe wurde bei einem Einsatzgemisch aus 50 g 2-Acetyl-6-methylnaphthalin, 300 g Essigsäure, 100 g Wasser und 7,5 g Mangan (II)-acetat wiederholt. Aus dem Reaktionsgemisch wurden durch Filtration 39,2 g Oxidationsprodukt isoliert. 20 g dieses Produktes wurden dann analog der in Beispiel 1 beschriebenen 2. Oxidationsstufe in einer Lösung von 2 g Kobalt (II)-acetat und 0,5 g Natriumbromid in 500 g Essigsäure bei 180°C und einem Gasaustritt von 2 l/min. oxidiert. Aus dem Reaktionsgemisch wurden dann 18,7 g 2,6-Naphthalindicarbonsäure mit einer Reinheit von 97,7% isoliert.

Beispiel 4

Analog Beispiel 3 wurde zunächst ein Gemisch aus 50 g 2-Acetyl-6-methylnaphthalin, 400 g Essigsäure und 10 g Mangan (II)-acetyl-acetonat bei 140°C oxidiert. Von den danach isolierten 31,7 g Oxidationsprodukt wurden 10 g in einer Lösung von 2 g Kobalt (II)-acetat und 0,5 g Natriumbromid in 500 g Essigsäure einer 2. Oxidation bei 190°C unterworfen. Danach wurden 9,6 g 2,6-Naphthalindicarbonsäure mit einer Reinheit von 97,7% erhalten.

Beispiel 5

Ein mit Rührer, Gaseinleitungsrohr, Temperaturfühler, Manometer und Druck-Rückflußkühler ausgestatteter, beheizbarer Autoklav aus Hastellog C wurde mit 25 g 6-Methyl-2-naphthoesäure (Reinheit 96,8%), 1.200 g 95 Gew.-%iger Essigsäure, 8 g Kobalt (II)-acetat-tetrahydrat und 8 g Natriumbromid beschickt, unter Stickstoff auf 185°C erhitzt und dann bei einem Druck von 25 bar mit einer nach der Entspannung gemessenen Gasaustrittsgeschwindigkeit von 2 l/min. Luft durch die Lösung geleitet. Der Reaktionsverlauf wurde durch kontinuierliche Messung des Sauerstoffgehaltes im Abgas kontrolliert. Nachdem hier der Sauerstoffgehalt auf 7% angestiegen war, wurde die Reaktion unterbrochen, dem entspannten Reaktionsgemisch bei unter 100°C nochmals 25 g 6-Methyl-2-naphthoesäure gleicher Qualität zugesetzet und in der zuvor beschriebenen Weise erneut oxidiert. Der Vorgang wurde dann weitere zweimal wiederholt, nach der letzten Zugabe der Methylnaphthoesäure wurde jedoch die Lufteinleitung bei 185°C so lange fortgesetzt, bis der Sauerstoffgehalt im Abgas einen Wert von 20% erreicht hatte. Das abgekühlte und entspannte Reaktionsgemisch wurde filtriert, der Filterkuchen mit 400 g Essigsäure gewaschen und getrocknet. Es resultierten 105,3 g (91,5% d. Th.) 2,6-Naphthalindicarbonsäure mit einer Reinheit von 97,8%.

Die mit dem Waschfiltrat vereinte Mutterlauge wurde destillativ auf ein Gewicht von 1.230 g mit einem Wassergehalt von 5 Gew.-% eingeengt, 25 g 6-Methyl-2-naphthoesäure der gensnnten Qualität und 2 g Natriumbromid zugesetzt und in der zuvor beschriebenen Weise zunächst diese und dann weitere dreimal je 25 g 6-Methyl-2-naphthoesäure oxidiert. Diesmal wurden 108,7 g (94,5% d. Th.) 2,6-Naphthalindicarbonsäure mit einer Reinheit von 97,7% erhalten.

Beispiel 6

Analog Beispiel 5 wurden zunächst 25 g und dann in 8 gleichen Portionen insgesamt weitere 188 g 6-Methyl-2-naphthoesäure (Reinheit 97,6%) in 1.200 g 97,5 Gew.-%iger Essigsäure unter den in Beispiel 1 genannten Katalysator- und Reaktionsbedingungen oxidiert. Es resultierten 230,8 g einer 97,9%igen 2,6-Naphthalindicarbonsäure (93,6% d. Th).

Beispiel 7

Der in Beispiel 5 beschriebene Versuch wurde mit 120 g 6-Methyl-2-naphthoesäure in 12 Portionen zu je 10 g wiederholt. Dabei wurden 131,6 g Naphthalindicarbonsäure einer Reinheit von 98,9% gewonnen (Ausbeute 96,5% d. Th.).

Beispiel 8

Beispiel 5 wurde wiederholt, jedoch mit nur 800 g 95 Gew.-%iger Essigsäure und 10 g 6-Methyl-2-naphthoesäure (Reinheit 96,2%) am Beginn und Zugabe von 100 g Ausgangsstoff in 400 g der Essigsäure in 20, im wesentlichen gleichen Portionen während der Reaktion. Es wurden 118,8 g Produkt erhalten. Reinheit 99,1%, Ausbeute 95,8% d. Th.

**Patentansprüche**

1. Verfahren zur Herstellung von 2,6 Naphthalindicarbonsäure, durch Oxidation eines 2-Acyl-6-alkylnaphthalins der Formel

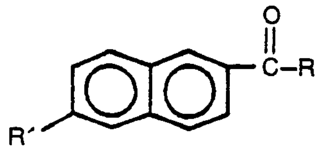

(I)

worin R und R' gleiche oder unterschiedliche, geradkettige oder verzweigte Alkylgruppen mit 1 bis 6 C-Atomen, wobei das in R'dem aromatischen Rest bzw. das in R der Carbonylgruppe nächststehende C-Atom zumindest noch 1 H-Atom tragen muß, bedeuten, in flüssiger Phase mit Sauerstoff bzw. einem Sauerstoff enthaltenden Gas, dadurch gekennzeichnet, daß das 2-Acyl-6-alkylnaphthalin in einer ersten Oxidationsstufe in Gegenwart einer im Reaktionsgemisch löslichen Manganverbindung als Katalysator in einer aliphatischen Carbonsäure als Lösungsmittel bzw. in einem eine aliphatische Carbonsäure enthaltenden Lösungsmittelgemisch, gegebenenfalls in Anwesenheit von Kobalt- oder Bromkatalysator, jedoch nicht in Anwesenheit von Kobalt- und Bromkatalystoren gleichzeitig, bei Temperaturen von 80 bis 180°C oxidiert wird und die enstehende 6-Alkyl-2-naphthoesäure in einer zweiten Oxidationsstufe in Gegenwart einer in Reaktionsgemisch löslichen Kobaltverbindung und einer Bromionen liefernden Komponente als Katalysator in einer aliphatischen Carbonsäure als Lösungsmittel bzw. in einem eine aliphatische Carbonsäure enthaltenden Lösungsmittelgemisch bei einer reaktionstemperatur von 150 bis 250°C und einem Druck von 5 bis 80 bar zum Produkt oxidiert wird.

2. Verfahren nach Anspruch 1, dadurch gekennzeichnet, daß in beiden Oxidationsstufen Essigsäure oder eine wässrig-verdünnte Essigsäure als Lösungsmittel verwendet wird.

3. Verfahren nach Anspruch 1 oder 2, dadurch gekennzeichnet, daß als 2-Acyl-6-alkylnaphthalin in der ersten Oxidationsstufe 2-Acetyl-6-methylnaphthalin eingesetzt wird.

4. Verfahren nach einem der Ansprüche 1 bis 3, dadurch gekennzeichnet, daß in der ersten Oxidationsstufe pro Gew.-Teil des 2-Acyl-6-alkylnaphthalins 0,001 bis 0,1 vorzugsweise 0,01 bis 0,05 Gew.-Teile Mangan in Form einer im Reaktionsgemisch löslichen Verbindung, vorzugsweise als Mangan (II)-acetat und/oder Mangan (II)-acetyl-acetonat eingesetzt werden.

5. Verfahren nach einem der Ansprüche 1 bis 4, dadurch gekennzeichnet, daß das 2-Acyl-6-alkylnaphthalin und das Lösungsmittel in Gewichtsverhältnis 1:2 bis 1:20, vorzugsweise 1:3 bis 1:10 eingesetzt werden.

6. Verfahren nach einem der Ansprüche 1 bis 5, dadurch gekennzeichnet, daß die erste Oxidationsstufe bei einer Temperatur von 100 bis 160°C und einem Druck von 5 bis 60 bar durchgeführt wird.

7. Verfahren nach einem der Ansprüche 1 bis 6, dadurch gekennzeichnet, daß das Oxidationsprodukt nach der ersten Oxidationsstufe durch eine Fest-Flüssig-Trennung isoliert und die Mutterlauge nach destillativer Abtrennung von Wasser, Ameisensäure und ggf. mit Waschfiltraten zusätzlich eingebrachten Lösungsmittel erneut als Reaktionsmedium für die Oxidation von 2-Acyl-6-alkylnaphthalin verwendet wird.

8. Verfahren nach einem der Ansprüche 1 bis 2, dadurch gekennzeichnet, daß pro Gew.-Teil des in der ersten Oxidationsstufe eingesetzten 2-Acyl-6-alkylnaphthalins bzw. des daraus entstandenen, isolierten und in der zweiten Oxidationsstufe eingesetzten Oxidationsproduktes dort 0,0005 bis 0,05, vorzugsweise 0,002 bis 0,02 Gew.-Teile Kobalt in Form einer in Reaktionsgemisch löslichen Kobaltverbindung und 0,001 bis 0,1, vorzugsweise 0,002 bis 0,04 Gew.-Teile Brom in Form einer unter den Reaktionsbedingungen Bromidionen liefernden Substanz eingesetzt werden.

9. Verfahren nach einem der vorangehenden Ansprüche, dadurch gekennzeichnet, das das nach der ersten Oxidationsstufe isolierte Oxidationsprodukt und das Lösungsmittel in der zweiten Oxidationsstufe im Gewichtsverhältnis 1:2 bis 1:50, vorzugsweise 1:4 bis 1:20 eingesetzt werden.

10. Verfahren nach einem der vorangehenden Ansprüche, dadurch gekennzeichnet, das die zweite Oxidationsstufe bei einer Temperatur von 150 bis 250°C, vorzugsweise bei 160 bis 220°C, und einem Druck von vorzugsweise von 10 bis 60 bar durchgeführt wird.

11. Verfahren nach einem der vorangehenden Ansprüche, dadurch gekennzeichnet, das die nach Abtrennung der 2,6-Naphthalindicarbonsäure durch eine Fest-Flüssig-Trennung des Reaktionsgemisches

nach der zweiten Oxidationsstufe erhaltene Mutterlauge nach destillativer Abtrennung des Reaktionswassers und ggf. mit Waschfiltraten eingebrachter Lösungsmittel erneut als Reaktionsmedium für die zweite Oxidationsstufe verwendet wird.

12. Verfahren nach einem der vorangehenden Ansprüche, dadurch gekennzeichnet, das der Ausgangsstoff während der Reaktion kontinuierlich oder in Portionen zugegeben wird.

13. Verfahren nach Anspruch 12, dadurch gekennzeichnet, daß insgesamt 0,1 bis 0,4 Gew.-Teile Ausgangsstoff pro Gew.-Teil Lösungsmittel eingesetzt werden.

**Revendications**

1. Procédé de préparation de l'acide naphthalène-2,6-dicarboxylique par oxidation d'un 2 acyl-6-alkyl naphthalène de formule:

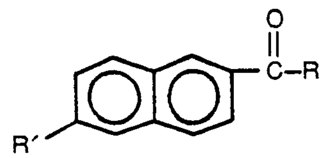

(I)

dans laquelle R et R' représentent les groupes alkyles identiques ou différents, linéaires ou ramifiés, ayant 1 à 6 atomes de carbone, l'atome de carbone le plus voisin de R' du reste aromatique ou du R du groupe carbonyle devant porter an moins encore un atome de H, en phase liquide, à l'aide d'oxygène ou d'un gaz contenant de l'oxygène, procédé caractérisé en ce qu'on oxyde le 2-acyl-6-alkyl-naphthalène, dans une première étape d'oxydation, en présence d'un composé du manganèse, soluble dans le mélange réactionnel, comme catalyseur dans un acide carboxylique aliphatique comme solvant ou dans un mélange de solvants contenant de l'acide carboxylique, éventuellment en présence d'un catalyseur au cobalt ou au brome, mais non pas en présence simultanée de catalyseurs au cobalt et au brome, à des températures de 80 à 180°C, et que l'on soumet l'acide 6-alkyl-2-naphthoïque résultant à une oxydation, dans une seconde étape d'oxydation, en présence d'un composé du cobalt et d'un composant fournissant des ions brome, solubles dans le mélange réactionnel, à titre de catalyseur, dans un acide carboxylique aliphatique comme solvant ou dans un mélange de solvants contenant un acide carboxylique aliphatique, à une température de réaction de 150 à 250°C et sous une pression de 5 à 80 bars, pour obtenir le produit.

2. Procédé selon la revendication 1, caractérisé en ce que, dans les deux étapes d'oxydation, on utilise comme solvant l'acide acétique ou un acide acétique aqueux dilué que l'eau.

3. Procédé selon la revendication 1 ou 2, caractérisé en ce qu'on utilise, comme 2-acyl-6-alkyl-naphtalène dans la première étape d'oxydation, le 2-acétyl-6-méthyl-naphthalène.

4. Procédé selon l'une des revendications 1 à 3, caractérisé en ce que, dans la première étape d'oxydation, on utilise par parties en poids du 2-acyl-6-alkyl-naphthalène 0,001 à 0,1, avantageusement 0,01 à 0,05 partie en poids de manganèse, sous forme d'un composé soluble dans le mélange réactionnel, avantageusement sous forme d'acétate de manganèse-(II) et/ou d'acétyl-acétonate de manganèse-(II).

5. Procédé selon l'une des revendications 1 à 4, caractérisé en ce qu'on utilise le 2-acyl-6-alkyl-naphthalène et le solvant selon un rapport pondéral de 1:2 à 1:20, avantageusement de 1:3 à 1:10.

6. Procédé selon l'une des revendications 1 à 5, caractérisé en ce qu'on conduit la première étape d'oxydation à une température de 100 à 160°C sous une pression de 5 à 60 bars.

7. Procédé selon l'une des revendications 1 à 6, caractérisé en ce qu'après la première étape d'oxydation, on insole le produit de l'oxydation par une séparation solide/liquide et, après avoir séparé par distillation l'eau, l'acide formique et éventuellement du solvant introduit en plus avec les filtrats de lavage, on utilise à nouveau la liqueur mère comme milieu de réaction pour l'oxydation du 2-acyl-6-alkyl-naphtalène.

8. Procédé selon l'une des revendications 1 à 2, caractérisé en ce que, par partie en poids du 2-acyl-6-alkyle-naphtalène, utilisé dans la première étape d'oxydation, ou du produit d'oxydation qui résulte de cette étape, qui a été isolé et qui est utilisé dans la seconde étape d'oxydation, on utilise dans cette seconde étape 0,0005 à 0,05, avantageusement 0,002 à 0,02 partie en poids de cobalt sous forme d'un composé de cobalt soluble dans le mélange réactionnel, et 0,001 à 0,1, avantageusement 0,002 à 0,04 partie en poids de brome, sous forme d'une substance fournissant des ions brome dans les conditions de réaction.

9. Procédé selon l'une des revendications precédentes, caractérisé en ce qu'on utilise le produit d'oxydation, isolé après la première étape d'oxydation, et le solvant dans la seconde étape d'oxydation selon un rapport pondéral allant de 1:2 à 1:50, avantageusement de 1:4 à 1:20.

10. Procédé selon l'une des revendications precédentes, caractérisé en ce qu'on conduit la seconde étape d'oxydation à une température de 150 à 250°C, avantageusement entre 160 et 220°C, et sous une pression qui est avantageusement de 10 à 60 bars.

11. Procédé selon l'une des revendications precédentes, caractérisé en ce que, après avoir séparé l'acide naphtalène-2,6-dicarboxylique, par une séparation solide/liquide du mélange réactionnel, on utilise à nouveau, comme milieu de réaction pour la seconde étape d'oxydation, la liqueur mère obtenue après la seconde étape d'oxydation, après en avoir séparé par distillation l'eau de réaction et éventuellement le solvant introduit avec les filtrats de lavage.

12. Procédé selon l'une des revendications precédentes, caractérisé en ce qu'on ajoute en continu ou par portions la substance de départ pendant la réaction.

13. Procédé selon la revendication 12, caractérisé en ce qu'on utilise au total 0,1 à 0,4 partie en poids de la substance de départ par partie en poids du solvant.

**Claims**

1. Process for the preparation of 2,6-naphthalene dicarboxylic acids by oxidation in the liquid phase with oxygen or an oxygen-containing gas of a 2-acyl-6-alkylnaphthalene of the formula

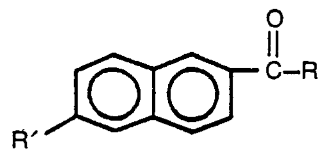

(I)

wherein R and R' signify the same or different straight-chain or branched alkyl groups with 1 to 6 C-atoms, wherein the C-atom standing next, in R', to the aromatic residue or, in R, to the carbonyl group must still carry at least 1 H-atom, characterised in that the 2-acyl-6-alkylnaphthalene is oxidised at temperatures of 80 to 180°C in a first oxidation step in the presence of a manganese compound soluble in the reaction mixture, as catalyst, in an aliphatic carboxylic acid, as solvent, or in a solvent mixture containing an aliphatic carboxylic acid, optionally in the presence of cobalt or bromine catalyst, although not in the presence of cobalt and bromine catalysts simultaneously, and the 6-alkyl-2-naphthoic acid is oxidised to form the product in a second oxidation step at a reaction temperature of 150 to 250°C and a pressure of 5 to 80 bar to form the product in a second oxidation step in the presence of a cobalt compound soluble in the reaction mixture and a component yielding bromine ions as catalyst in an aliphatic carboxylic acid as solvent or in an aliphatic carboxylic acid containing solvent mixture.

2. Process according to claim 1, characterised in that acetic acid or a water-diluted acetic acid is used as solvent in both oxidation steps.

3. Process according to claim 1 or 2, characterised in that 2-acetyl-6-methylnaphthalene is employed in the first oxidation step as 2-acyl-6-alkylnaphthalene.

4. Process according to one of claims 1 to 3, characterised in that 0.001 to 0.1, preferably 0.01 to 0.05 parts by weight of manganese in the form of a compound soluble in the reaction mixture, preferably as manganese (II)-acetate and/or manganese (II)-acetylacetonate are employed per part by weight of 2-acyl-6-alkylnaphthalene in the first oxidation step.

5. Process according to one of claims 1 to 4, characterised in that the 2-acyl-6-alkylnaphthalene and the solvent are employed in the weight ratio 1:2 to 1:20, preferably 1:3 to 1:10.

6. Process according to one of claims 1 to 3, characterised in that the first oxidation step is carried out at a temperature of 100 to 160°C and a pressure of 5 to 60 bar.

7. Process according to one of claims 1 to 6, characterised in that the oxidation product is isolated after the first oxidation step by a solid-liquid separation and the mother liquor is used again as reaction medium for the oxidation of 2-acyl-6-alkylnaphthalene, after distillative separation off of water, formic acid and possibly of solvent employed additionally along with washing filtrates.

8. Process according to one of claims 1 to 2, characterised in that 0.0005 to 0.05, preferably 0.002 to 0.02 parts by weight of cobalt in the form of a cobalt compound soluble in the reaction mixture and 0.001 to 0.1, preferably 0.002 to 0.04 parts by weight of bromine in the form of a substance yielding bromide ions under the reaction conditions are employed per part by weight of the 2-acyl-6-alkylnaphthalene employed in the first reaction step or the oxidation product being produced therefrom which is isolated and employed in the second oxidation step.

9. Process according to one of the foregoing claims, characterised in that the oxidation product isolated after the first oxidation step and the solvent are employed in the second oxidation step in the weight ratio of 1:2 to 1:50, preferably 1:4 to 1:20.

10. Process according to one of the foregoing claims, characterised in that the second oxidation step is carried out at a temperature of 150 to 250°C, preferably at 160 to 220°C, and a pressure of preferably from 10 to 60 bar.

11. Process according to one of the foregoing claims, characterised in that the mother liquor obtained after separation off of the 2,6-naphthalene dicarboxylic acid by a solid-liquid separation of the reaction mixture after the second oxidation step is used again as reaction medium for the second oxidation step after distillative separation off of the reaction water and possibly of solvent employed along with the washing filtrates.

12. Process according to one of the foregoing claims, characterised in that the starting material is added continuously or in portions during the reaction.

13. Process according to claim 12, characterised in that 0.1 to 0.4 parts by weight of starting material are employed in total per part by weight of solvent.